(19) Europäisches Patentamt
European Patent Office
Office européen des brevets

(11) Veröffentlichungsnummer: **0 491 329 A1**

(12) # EUROPÄISCHE PATENTANMELDUNG

(21) Anmeldenummer: **91121548.1**

(51) Int. Cl.[5]: **C07D 239/42**

(22) Anmeldetag: **16.12.91**

(30) Priorität: **18.12.90 CH 4015/90**
**04.03.91 CH 639/91**

(43) Veröffentlichungstag der Anmeldung:
**24.06.92 Patentblatt 92/26**

(84) Benannte Vertragsstaaten:
**AT BE CH DE ES FR GB IT LI NL SE**

(71) Anmelder: **LONZA AG**
**Gampel/Wallis Geschäftsleitung Basel**
**CH-4002 Basel(CH)**

(72) Erfinder: **Kuo, David L., Dr.**
**Bielastrasse 51**
**Brig (Kanton Wallis)(CH)**
Erfinder: **Voeffray, Robert, Dr. Chem.**
**Florastrasse 37**
**Basel(CH)**

(74) Vertreter: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40(DE)**

(54) **Verfahren zur Herstellung von Piperazinylpyrimidin-Derivaten.**

(57) Beschrieben wird ein neues Verfahren zur Herstellung von Piperazinylpyrimidin-Derivaten der allgemeinen Formel

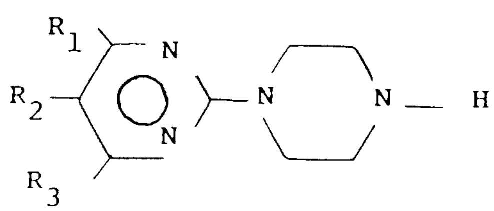

I

worin $R_1$, $R_2$ und $R_3$ gleich oder verschieden sind und ein Wasserstoffatom oder eine $C_1$-$C_4$-Alkylgruppe, verzweigt oder unverzweigt, bedeuten.
Diese Piperazinylpyrimidin-Derivate werden ausgehend von Piperazin oder dessen Hydrat sauer mit Cyanamid in ein Amidinsalz überführt und dann mit einer 1,3-Dicarbonylverbindung zum Endprodukt umgesetzt.

EP 0 491 329 A1

Die Erfindung betrifft ein neues Verfahren zur Herstellung von Piperazinylpyrimidin-Derivaten der allgemeinen Formel

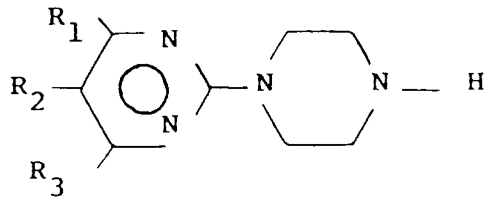

I

worin $R_1$, $R_2$ und $R_3$ gleich oder verschieden sind und ein Wasserstoffatom oder eine $C_1$-$C_4$-Alkylgruppe, verzweigt oder unverzweigt, bedeuten.

Die Piperazinylpyrimidin-Derivate der allgemeinen Formel I sind wichtige Zwischenprodukte zur Herstellung von pharmazeutischen Produkten wie beispielsweise zur Herstellung von 4-Methyl-2-piperazinylpyrimidin, welches den Blutzuckerspiegel senkt (EP-A 0 330 263, DE 33 21 969).

Verfahren zur Herstellung von Piperazinylpyrimidin-Derivaten sind in der EP-A 0 330 263 beschrieben. Bei diesen Verfahren wird ein chloriertes Pyrimidin-Derivat mit einem Piperazin-Derivat in ein Piperazinylpyrimidin-Derivat überführt. Nachteile dieser Verfahren sind, dass die Synthesen über mehrere Stufen erfolgen und die Produkte in schlechter Ausbeute erhalten werden.

Aufgabe der vorliegenden Erfindung war es, diese Nachteile zu beseitigen und ein einfaches und wirtschaftliches Verfahren zur Herstellung von Piperazinylpyrimidin-Derivaten zur Verfügung zu stellen.

Diese Aufgabe wurde gemäss Patentanspruch 1 gelöst.

Das Verfahren zur Herstellung von Piperazinylpyrimidin-Derivaten der allgemeinen Formel

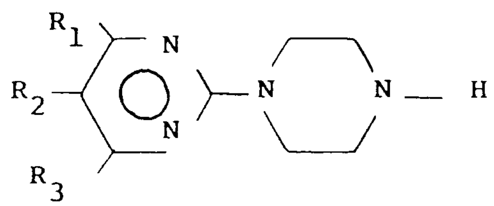

I

worin $R_1$, $R_2$, und $R_3$ gleich oder verschieden sind und ein Wasserstoffatom oder eine $C_1$-$C_4$-Alkylgruppe, verzweigt oder unverzweigt, bedeuten, wird derart durchgeführt, dass man in der ersten Stufe Piperazin oder dessen Hydrat der Formel

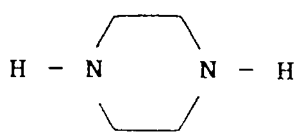

II

sauer mit Cyanamid auf bekannte Weise, gemäss JP 51-39680, zu einem Amidinsalz der Formel

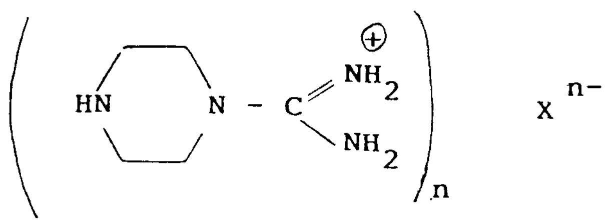

III

worin X ein Salzanion bedeutet und n der Wertigkeit dieses Salzanions entspricht, umsetzt, dieses gegebenenfalls isoliert und dann in der zweiten Stufe mit einer 1,3-Dicarbonyl-Verbindung oder einen Äquivalent derselben zum Endprodukt umsetzt.

Zweckmässig wird die Umsetzung in der ersten Stufe, gemäss der JP 51-39680, mit 1 bis 1,5 mol Cyanamid, Vorzugsweise mit 1,1 mol Cyanamid, bezogen auf 1 mol Piperazin beziehungsweise dessen Hydrat,durchgeführt.

Als Lösungsmittel wird zweckmässig das selbe Lösungsmittel wie in der zweiten Stufe verwendet.

Zweckmässig wird die Umsetzung in der ersten Stufe bei einer Temperatur von 40 bis 60°C durchgeführt.

Zweckmässig wird die Umsetzung in der ersten Stufe in Gegenwart einer Mineralsäure durchgeführt. Als Mineralsäure können beispielsweise Schwefelsäure, Salzsäure oder Phosphorsäure angewendet werden. Dementsprechend bedeutet dann $X^{n-}$ im Amidinsalz Chlorid, Sulfat oder Phosphat. Insbesondere wird als Mineralsäure konzentrierte Schwefelsäure angewendet. Vorzugsweise werden die Mineralsäure und das Piperazin oder dessen Hydrat für die erste Stufe aequimolar eingesetzt.

Nach einer üblichen Reaktionszeit von 1 bis 2 Stunden wird dann gegebenenfalls das Amidinsalz nach üblichen Aufarbeitungsmethoden, z.B. durch Einengen, erhalten, oder das ganze Reaktionsgemisch wird direkt für die zweite Stufe eingesetzt.

Die zweite Stufe, die Umsetzung des Amidinsalzes zum Endprodukt, erfolgt mit einer 1,3-Dicarbonyl-Verbindung oder einem Äquivalent derselben. Geeignete Vertreter von 1,3-Dicarbonyl-Verbindungen bzw. deren Äquivalenten sind:
Verbindungen der allgemeinen Formel

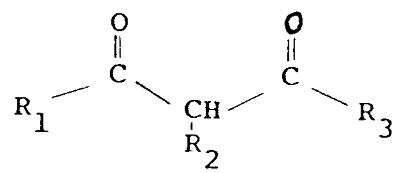

IV

worin $R_1$, $R_2$ und $R_3$ gleich oder verschieden sind und ein Wasserstoffatom oder eine $C_1$-$C_4$-Alkylgruppe, verzweigt oder unverzweigt, bedeuten oder Tetra($C_1$-$C_4$)alkoxypropane der allgemeinen Formel

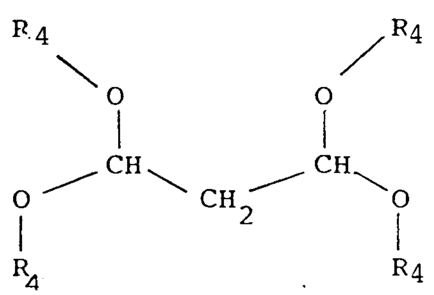

V

worin $R_4$ eine $C_1$-$C_4$-Alkylgruppe, verzweigt oder unverzweigt, bedeutet.

Umsetzung mit einer Dicarbonylverbindung der allgemeinen Formel IV

Bevorzugte Verbindungen der Formel IV sind:
2,4-Octandion ($R_1$ = Butyl, $R_2$ = H, $R_3$ = Methyl)
Acetylaceton ($R_1$ = Methyl, $R_2$ = H, $R_3$ = Methyl)
3-Methyl-2,4-pentandion ($R_1$ = $R_2$ = $R_3$ = Methyl)
4-Hydroxy-3-methyl-3-buten-2-on ($R_1$ = $R_2$ = Methyl, $R_3$ = H)

Die Herstellung von 2,4-Octandion als Vertreter der Verbindungen der allgemeinen Formel IV erfolgt, ausgehend von Acetylaceton, durch Reaktion mit Natrium, flüssigem Ammoniak und Propylbromid (R. Hausen et al., Org.Synthesis, 1967, 47, S.92) und die Herstellung von 4-Hydroxy-3-methyl-3-buten-2-on als Vertreter der allgemeinen Formel IV durch Umsetzung von 2-Butanon mit Methylformiat und metallischem Natrium (Tracy et al., J.Org.Chem., 1941, S.63). Die übrigen Dicarbonylverbindungen sind kommerziell erhältlich.

Zweckmässig wird die Umsetzung mit der 1,3-Dicarbonyl-Verbindung der allgemeinen Formel IV in der zweiten Stufe mit 0,5 bis 3 mol Dicarbonylverbindung, vorzugsweise mit 0,5 bis 2 mol, bezogen auf 1 mol

Amidinsalz, durchgeführt, bei einer zweckmässigen Temperatur von 20 bis 100 °C, vorzugsweise bei einer Temperatur von 65 bis 80 °C.

Zweckmässig wird die Umsetzung mit der Verbindung der allgemeinen Formel IV in der zweiten Stufe in einem polaren Lösungsmittel durchgeführt.
Als polare Lösungsmittel können niedrig siedende Alkohole dienen, wie beispielsweise Methanol, Ethanol oder Propanol. Vorzugsweise wird als Lösungsmittel Methanol angewendet.

Die Umsetzung in der zweiten Stufe, die mit der Verbindung der allgemeinen Formel IV, wird in Gegenwart einer Base durchgeführt. Als Base kann ein Alkoholat angewendet werden, wie beispielsweise Natriummethanolat, Natriumethanolat, Natriumpropanolat, Natriumbutanolat oder Kalium-tert.-butanolat.
Vorzugsweise wird Natriummethanolat angewendet.

Vorzugsweise wird die Base in einer Menge von 1 bis 4 mol, bezogen auf 1 mol Dicarbonylverbindung, eingesetzt.

Nach einer üblichen Reaktionszeit von 2 bis 10 Stunden wird dann das Endprodukt nach üblichen Aufarbeitungsmethoden,z.B. durch Extraktion oder Säulenchromatographie, isoliert.

Umsetzung mit einem Tetra($C_1$-$C_4$)alkoxypropan der Formel V

Als bevorzugter Vertreter der Formel V wird Tetramethoxypropan eingesetzt.

Zweckmässig wird die Umsetzung in der zweiten Stufe mit 0,25 bis 2 mol Tetra($C_1$-$C_4$)-alkoxypropan, vorzugsweise mit 1 mol, bezogen auf 1 mol Amidinsalz, durchgeführt, bei einer zweckmässigen Temperatur von 20 bis 100 °C, vorzugsweise bei einer Temperatur von 60 bis 80 °C.

Zweckmässig wird die Umsetzung in der zweiten Stufe mit einem Tetra($C_1$-$C_4$)alkoxypropan in Gegenwart einer wässrigen Mineralsäure durchgeführt.

Als Mineralsäuren können beispielsweise Schwefelsäure, Salzsäure oder Phosphorsäure angewendet werden.
Zweckmässig wird als wässrige Mineralsäure eine 30 bis 60%ige Mineralsäure angewendet.

Die wässrige Mineralsäure kann in einer Menge von 4 bis 10 mol, bezogen auf 1 mol Tetra($C_1$-$C_4$)-alkoxypropan, angewendet werden.
Vorzugsweise wird eine 30 bis 60%ige Schwefelsäure in einer Menge von 4 bis 10 mol, bezogen auf 1 mol Tetra($C_1$-$C_4$)alkoxypropan, angewendet.
Nach einer üblichen Reaktionszeit von 1 bis 24 Stunden wird dann das Endprodukt nach üblichen Aufarbeitungsmethoden, z.B. durch Extraktion und anschliessende Destillation, gewonnen.

Vorzugsweise wird die ganze Umsetzung in einem Eintopfverfahren zum Endprodukt durchgeführt.

Vorzugsweise werden als Endprodukte 2-(1-Piperazinyl)pyrimidin, 6-(1-Butyl)-4-methyl-2-(1-piperazinyl)-pyrimidin, 4,6-Dimethyl-2-(1-piperazinyl)pyrimidin, 4,5-Dimethyl-2-(1-piperazinyl)pyrimidin und 4,5,6-Trimethyl-2-(1-piperazinyl)pyrimidin erhalten.

Diese Piperazinylpyrimidin-Derivate können beispielsweise mit Methyljodid oder Isopropylbromid in die entsprechenden alkylierten Piperazinylpyrimidin-Derivate (EP 115 714) überführt werden, die, wie eingangs erwähnt, wichtige Zwischenprodukte von pharmazeutischen Produkten sind, wie beispielsweise 4-Methyl-2-piperazinylpyrimidin, zur Senkung des Blutzuckerspiegels.

Beispiel 1

Herstellung von 2,4-Octandion (nicht erfindungsgemäss)

Zu einer flüssigen Ammoniak-Lösung (400 ml) wurde metallisches Natrium (0,3 g, 13 mmol) hinzugegeben.
Nachdem die Mischung blau gefärbt war, wurde Eisennitrathydrat (0,13 g, 0,3 mmol) hinzugefügt und anschliessend nochmals metallisches Natrium (12,6 g, 0,55 mol). Nach Beendigung der $NaNH_2$-Bildung wurde eine Lösung von Acetylaceton (30 g, 299 mmol) in Ethylether (20 ml) innerhalb von 10 min bei -78 °C zugegeben.
Nach 20 min wurde Propylbromid (28,2 g, 299,6 mmol) tropfenweise innerhalb von 25 min zugegeben.
Nach weiterem Rühren während 50 min wurde Ethylether (100 ml) hinzugefügt und der Ammoniak abgedampft. Nach ca. 12 h wurde die Mischung mit Ethylether 3 mal (je 50 ml) extrahiert, und die vereinigten organischen Phasen wurden mit einer NaCl-Lösung 2 mal (je 50 ml) gewaschen und über $Na_2SO_4$ getrocknet. Nach Entfernen des Lösungsmittels wurden 33 g 2,4-Octandion erhalten.
Nach Vakuum-Destillation (13,5 mbar, 78 °C), wurden 22,59 g 2,4-Octandion, entsprechend einer Ausbeute von 52,9%, als farbloses Oel erhalten.

Beispiel 2

Herstellung von 4-Hydroxy-3-methyl-3-buten-2-on (nicht erfindungsgemäss)

Zu einer Lösung von 2-Butanon (50 g, 0,69 mol) und Methylformiat (41,9 g, 0,69 mol) in Diethylether wurde bei 0 ° C metallisches Natrium (15,87 g, 0,69 mol) in einem Zeitraum von 4 h hinzugegeben. Die resultierende gelbe Mischung wurde 14 h lang bei 20 ° C gerührt.
Das gebildete Natriumsalz wurde filtriert und mit zuvor getrocknetem Diethylether gewaschen.
Anschliessend wurde das Natriumsalz in kaltem $H_2O$ gelöst, und die dunkelrote Flüssigkeit wurde mit 25%-iger Schwefelsäure (150 ml) bis zu einem pH-Wert von ca. 3,7 angesäuert. Dann wurde 5 mal mit Ethylether (100 ml) extrahiert, die vereinigten organischen Phasen 1 mal mit $H_2O$ (50 ml) und 1 mal mit NaCl-Lösung (50 ml) gewaschen und anschliessend über $Na_2SO_4$ getrocknet.
Nach Entfernen des Lösungsmittels im Vakuum wurden 39,59 g 4-Hydroxy-3-methyl-3-buten-2-on, entsprechend einer Ausbeute von 57%, erhalten.

Beispiel 3

Herstellung von 2-(1-Piperazinyl)-amidinsulfat

Zu Piperazinhexahydrat (552 g, 2,78 mol) wurde innerhalb von 10 min 95,6%-ige Schwefelsäure (74,5 g, 0,72 mol) hinzugegeben. Diese Mischung wurde auf 50 ° C erwärmt, und dann wurde eine 25%-ige wässrige Lösung von Cyanamid (484 g, 2,88 mol), innerhalb von 2 h langsam zugetropft.
Nach weiterem Rühren für 2 h bei 50 ° C wurde innerhalb von 10 min 95,6%-ige Schwefelsäure (0,47 mol) hinzugefügt und das Ganze auf 63 ° C erwärmt.
Dann wurde die Mischung bis zur Trockne eingeengt, und zu dieser klebrigen Masse (601 g) wurde abs. Methanol (800 ml) hinzugefügt und 1 h kühlgestellt.
Anschliessend wurde die Lösung filtriert, mit kaltem abs. Methanol 2 mal (100 ml) gewaschen.
Nach Trocknen im Vakuum wurden 328 g 2-(1-Piperazinyl)-amidinsulfat, entsprechend einer Ausbeute von 77,3%, erhalten. Die Mutterlauge wurde 2 Tage lang gekühlt, anschliessend filtriert, mit abs. Methanol 2 mal (50 ml) gewaschen und unter Vakuum getrocknet, wobei 43,3 g 2-(1-Piperazinyl)-amidinsulfat, entsprechend einer Ausbeute von 10,2%,erhalten wurden. Nach Vereinigung dieser beiden Fraktionen betrug die Gesamtausbeute 87,6%, bezogen auf das Ausgangsprodukt.

Beispiel 4

Verfahren zur Herstellung von 2-(1-Piperazinyl)pyrimidin

Zu 2-(1-Piperazinyl)-amidinsulfat (5,2 g, 14,6 mmol) wurde 50%-ige Schwefelsäure (21,5 g, 109,5 mmol) hinzugegeben und die Mischung auf 70 ° C erwärmt.
Anschliessend wurde langsam Tetramethoxypropan (2,47 ml, 14,6 mmol) innerhalb von 2 h hinzugegeben.
Nach 5 h bei 70 ° C wurde die Mischung über Nacht auf 20 ° C abgekühlt. Dann wurde bei 0 ° C $H_2O$ (20 ml) und $CH_2Cl_2$ (40 ml) hinzugegeben und der pH der Lösung auf pH 14 mit 20%-iger NaOH (50 ml) eingestellt.
Das Gemisch wurde 5 mal mit $CH_2Cl_2$ (50 ml) extrahiert, und die vereinigten organischen Phasen wurden über $Na_2SO_4$ getrocknet; das Lösungsmittel wurde im Vakuum abgedampft, wobei 2,08 g Roh-Produkt, entsprechend einer Ausbeute von 86,6%, erhalten wurden.
Nach Destillation bei 150-175 ° C (0,5 mbar) wurden 1,37 g Produkt, entsprechend einer Ausbeute von 57%, bezogen auf eingesetztes Amidinsulfat, erhalten.

Beispiel 5

Herstellung von 6-(1-Butyl)-4-methyl-2-(1-piperazinyl)pyrimidin

Zu einer Lösung von 2-(1-Piperazinyl)amidinsulfat (10,5 g, 29,6 mmol) in abs. Methanol (20 ml) wurde Natriummethanolat (12,1 ml, 63,4 mmol) hinzugegeben.
Anschliessend wurde die Mischung 10 min. lang bei 80 ° C am Rückfluss erwärmt.
Danach wurde 2,4-Octandion (3,0 g, 21,1 mmol) innerhalb von 1,5 h hinzugegeben.
Nach beendeter Reaktion wurde die Mischung 12 h lang bei 80 ° C gerührt, abgekühlt und anschliessend 5

mal mit $CHCl_3$ (20 ml) extrahiert; dann wurden die vereinigten organischen Phasen 1 mal mit $H_2O$ (20 ml) gewaschen und über $Na_2SO_4$ getrocknet. Nach Entfernen des Lösungsmittels im Vakuum wurde ein Rückstand von 2,5 g erhalten, und nach Destillation (0-180 °C, 0,5 mbar) wurden 2,1 g Produkt, entsprechend einer Ausbeute von 42%, bezogen auf eingesetztes Amidinsulfat, als farbloses Oel erhalten.

Beispiel 6

Herstellung von 4,6-Dimethyl-2-(1-piperazinyl)pyrimidin

Zu einer Lösung von 2-(1-Piperazinyl)amidinsulfat (10 g, 28,2 mol) in abs. Methanol (20 ml) wurde Natriummethanolat (10,6 g, 58,8 mmol) hinzugegeben.
Anschliessend wurde die Mischung 10 min lang bei 80 °C am Rückfluss erwärmt. Dann wurde Acetylaceton (2,35 g, 23,5 mmol) innerhalb von 70 min hinzugegeben.
Nach beendeter Reaktion wurde die Mischung 15 h lang bei 80 °C gerührt, abgekühlt und anschliessend 4 mal mit $CHCl_3$ (25 ml) extrahiert; dann wurden die vereinigten organischen Phasen 1 mal mit $H_2O$ (20 ml) gewaschen und über $Na_2SO_4$ getrocknet. Nach Entfernen des Lösungsmittels im Vakuum wurde der Rückstand (3,1 g) destilliert (110-205 °C, 0,49 mbar), und es wurden 2,22 g Produkt, entsprechend einer Ausbeute von 49%, bezogen auf eingesetztes Amidinsulfat, als farbloses Oel erhalten.

Beispiel 7

Herstellung von 4,5-Dimethyl-2-(1-piperazinyl)pyrimidin

Zu einer Lösung von 2-(1-Piperazinyl)amidinsulfat (7 g, 20 mmol) in abs. Methanol (15 ml) wurde Natriummethanolat (11,1 g, 60 mmol) hinzugegeben.
Die Mischung wurde 10 min lang am Rückfluss auf 80 °C erhitzt. Anschliessend wurde über einen Zeitraum von 1,5 h 4-Hydroxy-3-methyl-3-buten-2-on (2 g, 20 mmol) hinzugegeben.
Nach beendeter Reaktion wurde die Mischung weitere 6 h bei 80 °C gerührt, abgekühlt und anschliessend 5 mal mit $CHCl_3$ (20 ml) extrahiert.
Die vereinigten organischen Phasen wurden 2 mal mit Wasser (50 ml) gewaschen und über $Na_2SO_4$ getrocknet.
Nach Entfernen des Lösungsmittels im Vakuum wurden 1,39 g Rückstand erhalten. Dieser wurde mittels Säulenchromatographie gereinigt (gefüllt mit Silica-Gel (Fluka, 230-440 mesh) und Methanol/Dichlormethan im Verhältnis 5:1 als Laufmittel). Danach wurden 0,5 g 4,5-Dimethyl-3-(1-piperazinyl)pyrimidin, entsprechend einer Ausbeute von 13%, bezogen auf eingesetztes Amidinsulfat, als farbloses Oel erhalten.

Beispiel 8

Herstellung von 4,5,6-Trimethyl-2-(1-piperazinyl)-pyrimidin

Zu einer Lösung von 2-(1-Piperazinyl)amidinsulfat (11,2 g, 31,6 mmol) in abs. Methanol (20 ml) wurde Natriummethanolat (14,6 g, 78,9 mmol) hinzugegeben.
Die Mischung wurde 20 min lang am Rückfluss auf 80 °C erhitzt, und dann wurde über einen Zeitraum von 2,5 h 3-Methyl-2,4-pentandion (3 g, 26,3 mmol) hinzugegeben. Nach beendeter Reaktion wurde die Mischung für weitere 3 h bei 80 °C gerührt, abgekühlt und anschliessend 5 mal mit $CHCl_3$ (20 ml) extrahiert. Die vereinigten organischen Phasen wurden 1 mal mit $H_2O$ (20 ml) gewaschen und über $Na_2SO_4$ getrocknet.
Nach Entfernung des Lösungsmittels im Vakuum wurde ein hellbrauner Rückstand (2,2 g) erhalten, der über Säulenchromatographie (Silica-Gel (Fluka 220-440 mesh) und Methanol/ Dichlormethan im Verhältnis 5:1 als Laufmittel) gereinigt wurde. Es wurden 1,99 g 4,5,6-Trimethyl-2-(1-piperazinyl)pyrimidin, entsprechend einer Ausbeute von 35%, bezogen auf eingesetztes Amidinsulfat, als farbloses Oel erhalten.

Beispiel 9

Herstellung von 4-Methyl-2-(1-(4-methylpiperazinyl))pyrimidin (nicht erfindungsgemäss)

Zu einer Lösung von 4-Methyl-2-(1-piperazinyl)pyrimidin (2,0 g, 11,2 mmol) in getrocknetem Dimethylformamid (15 ml) wurde unter Argon-Atmosphäre Natriumhydrid (0,4 g, 13,5 mmol) hinzugegeben. Die

Mischung wurde für weitere 55 min bei 20 ° C gerührt, und anschliessend wurde Methyljodid (1,75 g, 12,4 mmol) innerhalb von 10 min tropfenweise hinzugegeben. Die Mischung wurde für weitere 1,5 h gerührt, dann wurde vorsichtig $H_2O$ (5 ml) hinzugegeben. Nach 20 min wurde die Mischung 3 mal mit $CH_2Cl_2$ (15 ml) extrahiert, und die vereinigten organischen Phasen wurden 1 mal mit $H_2O$ (20 ml) gewaschen, dann über Natriumsulfat getrocknet.
Nach Entfernen des Lösungsmittels im Vakuum wurden 2,1 g gelbes Oel erhalten, welches über Säulenchromatographie (Silica-Gel, Laufmittel: Methanol/Dichlormethan im Verhältnis 1:1) gereinigt.
Es wurden 1,53 g 4-Methyl-2-(1-(4-methylpiperazinyl))pyrimidin, entsprechend einer Ausbeute von 71,2%, bezogen auf eingesetztes 4-Methyl-2-(1-piperazinyl)pyrimidin, als gelbes Oel erhalten.

## Patentansprüche

1. Verfahren zur Herstellung von Piperazinylpyrimidin-Derivaten der allgemeinen Formel

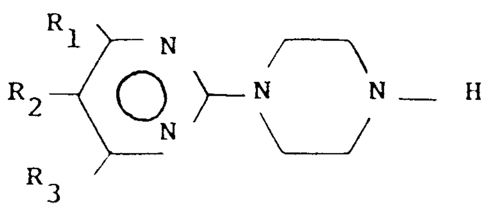

I

worin $R_1$, $R_2$ und $R_3$ gleich oder verschieden sind und ein Wasserstoffatom oder eine $C_1$-$C_4$-Alkylgruppe, verzweigt oder unverzweigt, bedeuten, dadurch gekennzeichnet, dass man in der ersten Stufe ein Piperazin oder dessen Hydrat der Formel

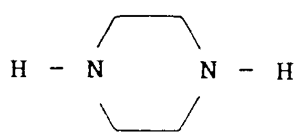

II

sauer mit Cyanamid in ein Amidinsalz der Formel

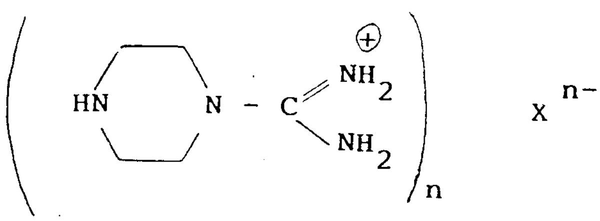

III

worin X ein Salzanion bedeutet und n der Wertigkeit dieses Salzanions entspricht, umsetzt, dieses gegebenenfalls isoliert und dann in der zweiten Stufe mit einer 1,3-Dicarbonyl-Verbindung oder einem Äquivalent derselben zum Endprodukt umsetzt.

2. Verfahren nach Patentanspruch 1, dadurch gekennzeichnet, dass man die Umsetzung in der ersten Stufe mit 1 bis 1,5 mol Cyanamid, bezogen auf 1 mol Piperazin, durchführt.

3. Verfahren nach mindestens einem der Patentansprüche 1 und 2, dadurch gekennzeichnet, dass man die Umsetzung in der ersten Stufe in Gegenwart einer Mineralsäure durchführt.

4. Verfahren nach mindestens einem der Patentansprüche 1 bis 3, dadurch gekennzeichnet, dass man die Umsetzung in der ersten Stufe bei einer Temperatur von 40 bis 60 ° C durchführt.

5. Verfahren nach mindestens einem der Patentansprüche 1 bis 4, dadurch gekennzeichnet, dass man in der zweiten Stufe als 1,3-Dicarbonyl-Verbindung eine Verbindung der allgemeinen Formel

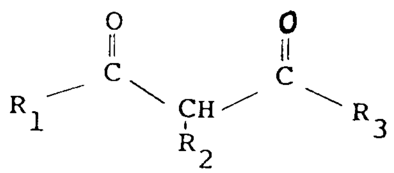

IV

worin $R_1$, $R_2$ und $R_3$ gleich oder verschieden sind und ein Wasserstoffatom oder eine $C_1$-$C_4$-Alkylgruppe, verzweigt oder unverzweigt, bedeuten, anwendet.

6. Verfahren nach mindestens einem der Patentansprüche 1 bis 4, dadurch gekennzeichnet, dass man in der zweiten Stufe als Äquivalent einer 1,3-Dicarbonyl-Verbindung ein Tetra($C_1$-$C_4$)alkoxypropan der allgemeinen Formel

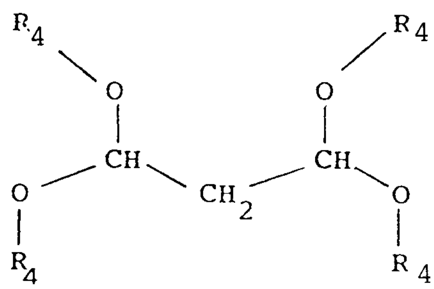

V

worin $R_4$ eine $C_1$-$C_4$-Alkylgruppe, verzweigt oder unverzweigt, bedeutet, anwendet.

7. Verfahren nach Patentanspruch 5, dadurch gekennzeichnet, dass man in der zweiten Stufe als Dicarbonylverbindung 2,4-Octandion, Acetylaceton, 3-Methyl-2,4-pentandion oder 4-Hydroxy-3-methyl-3-buten-2-on anwendet.

8. Verfahren nach mindestens einem der Patentansprüche 5 und 7 dadurch gekennzeichnet, dass man die zweite Stufe, die Umsetzung mit einer Dicarbonylverbindung IV, in Gegenwart einer Base in einem polaren Lösungsmittel durchführt.

9. Verfahren nach mindestens einem der Patentansprüche 5 und 7 bis 8, dadurch gekennzeichnet, dass man die zweite Stufe, die Umsetzung mit einer Dicarbonylverbindung IV, in Gegenwart eines Alkoholats als Base durchführt.

10. Verfahren nach Patentanspruch 6, dadurch gekennzeichnet, dass man in der zweiten Stufe als Tetra-($C_1$-$C_4$)alkoxypropan Tetramethoxypropan anwendet.

11. Verfahren nach mindestens einem der Patentansprüche 6 und 10, dadurch gekennzeichnet, dass man die zweite Stufe, die Umsetzung mit einem Tetra($C_1$-$C_4$)alkoxypropan, in Gegenwart einer wässrigen Mineralsäure durchführt.

12. Verfahren nach mindestens einem der Patentansprüche 5 bis 11, dadurch gekennzeichnet, dass man die Umsetzung in der zweiten Stufe bei einer Temperatur von 20 bis 100 °C durchführt.

Europäisches Patentamt

# EUROPÄISCHER RECHERCHENBERICHT

Nummer der Anmeldung

EP 91 12 1548

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | EP-A-0 129 128 (TROPONWERKE)<br>* Ansprüche 11-15 *<br>----- | 1-4 | C07D239/42 |
| | | | RECHERCHIERTE SACHGEBIETE (Int. Cl.5 ) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 19 MAERZ 1992 | FRANCOIS J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)